# EUROPEAN PATENT SPECIFICATION

(11) **EP 2 939 531 B1**
(45) Date of publication and mention of the grant of the patent: **01.03.2017**
(21) Application number: 14170609.3
(22) Date of filing: 30.05.2014
(51) Int. Cl.: A01H 4/00, A01G 9/08

(54) **Automatic dispensing apparatus**
Automatische Ausgabevorrichtung
Appareil de distribution automatique

(30) Priority: 29.04.2014 TW 103115407
(43) Date of publication of application: 04.11.2015
(73) Proprietor: Green Culture Biotechnology Co., Ltd., 435 Taichung City (TW)
(72) Inventor: Ho, Hung-Ming, 435 Taichung City (TW)
(74) Representative: Straus, Alexander

(56) References cited:
- EP-A2- 0 132 414
- WO-A1-2009/042099
- WO-A2-2008/013933
- US-A- 4 910 146

## Description

### BACKGROUND OF THE INVENTION

### 1. Field of the Invention

The present invention relates generally to a plant tissue culture technology and more particularly, to an automatic dispensing apparatus for a plant culture medium.

### 2. Description of the Related Art

Among the plant tissue culture technology, the most common method is to pour a culture medium containing plant cultures into a storage tank for agitation and after the agitation, a filling tube fills a plurality of culture containers with the culture medium, respectively, for growth cultivation. After each filling operation is completed, the operator needs to sterilize and clean the storage tank and the filling tube to prevent the culture medium from contamination next time.

However, the whole operation of aforesaid prior art is manually completed, so instability is subject to occurrence in the process of filling the culture containers with the culture medium and may lead to adverse influence on the quality of the plants cultivated. Besides, as far as the plant industry in need of mass production for higher economic value is concerned, the aforesaid prior art may lead to worse production efficiency and further lower competitivity. In order to overcome this potential disadvantage, automatic dispensing apparatuses for plant culture medium were developed, see for example WO 2009/042099.

### SUMMARY OF THE INVENTION

The primary objective of the present invention is to provide an automatic dispensing apparatus which can function as sterilization and cleaning and enhance production efficiency.

The foregoing objective of the present invention is attained by the automatic dispensing apparatus formed of two storage tanks, a control device, a filling device, and a dispensing device. Each of the storage tanks is to store a plant culture medium. The control device includes a switcher, a first feeding tube, a second feeding tube, a discharging tube, and a waste exhaust tube. The first and second feeding tubes are connected between the switcher and one of the storage tanks and between the other storage tank and the switcher, respectively. Each of the discharging tube and the waste exhaust tube includes an end connected with the switcher. When the switcher is located at a first mode, the first feeding tube, the second feeding tube, and the waste exhaust tube do not communicate with one another and one of the first and second feeding tubes communicates with the discharging tube, so the plant culture medium can be conveyed to the discharging tube through one of the first and second feeding tubes from the corresponsive storage tank. When the switcher is located at a second mode, the first feeding tube, the second feeding tube, and the discharging tube do not communicate with one another and one of the first and second feeding tubes communicates with the waste exhaust tube to allow a cleaning fluid to exhaust through the waste exhaust tube via one of the first and second feeding tubes from the corresponsive storage tank. The filling device s connected with the discharging tube for filling at least one culture container with the plant culture medium. The dispensing device is mounted adjacent to the filing device and includes a container carrier, an accommodating platform, and a conveying platform. The conveying platform is mounted in a way that the conveying platform can reciprocate between the filling device, the container carrier, and the accommodating platform for conveying the culture container to the filling device from the container carrier for the filing operation of the plant culture medium and then conveying the culture container to the accommodating platform after the filling operation.

In light of the structure mentioned above, the automatic dispensing apparatus can apply the filling and dispensing operations to at least two different plant culture media and the switcher can quickly and conveniently complete sterilization and cleaning.

### BRIEF DESCRIPTION OF THE DRAWINGS

FIG. 1 is a block diagram of a preferred embodiment of the present invention, illustrating that steam is infused into the storage tank for sterilization.
FIG. 2 is another block diagram of the preferred embodiment of the present invention, illustrating that cooling water is infused into a storage tank for cleaning.
FIG. 3 is another block diagram of the preferred embodiment of the present invention, illustrating that a plant culture medium is infused into the storage tank.
FIG. 4 is a perspective view of a control device in accordance with the preferred embodiment of the present invention.
FIG. 5 is a front view of a dispensing device in accordance with the preferred embodiment of the present invention.
FIG. 6 is similar to FIG. 5, illustrating that a container catcher catches a chassis and a top cover of a culture container.
FIG. 7 is a top view of a dispensing device, illustrating that the container catcher is moved toward a conveying platform via an X-axis slide base.
FIG. 8 is similar to FIG. 7, illustrating that the chassis of the culture container is engaged with a first engagement portion of the conveying device.

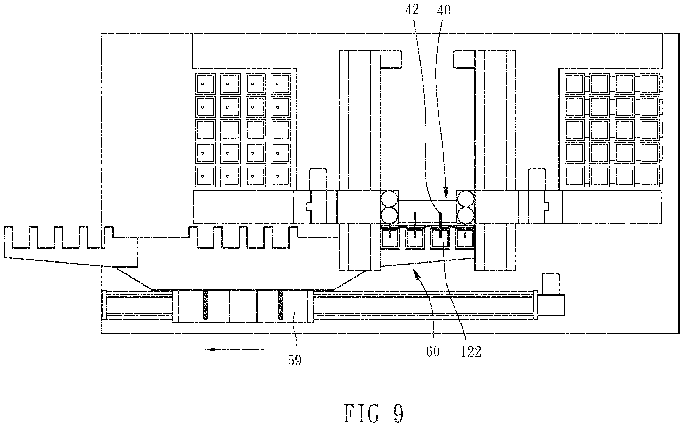

FIG. 9 is similar to FIG. 8, illustrating that the conveying platform conveys the chassis of the culture container to a position in front of the filling device.
FIG. 10 is similar to FIG. 9, illustrating that the first engagement portion of the conveying platform disengages from the chassis of the culture container.
FIG. 11 is similar to FIG. 10, illustrating that a second engagement portion is engaged with the chassis of the culture container.
FIG. 12 is similar to FIG. 11, illustrating that the conveying platform conveys the chassis of the culture container to a position in front of the top cover.
FIG. 13 is similar to FIG. 12, illustrating that the second engagement portion is engaged with the chassis of the culture container.
FIG. 14 is similar to FIG. 13, illustrating that the conveying platform conveys the culture container to the accommodating platform.

### DETAILED DESCRIPTION OF PREFERRED EMBODIMENTS

Structural features and desired effects of the present invention will become more fully understood by reference to a preferred embodiment given hereunder. However, it is to be understood that the embodiment is given by way of illustration only, thus being not limitative of the claim scope of the present invention.

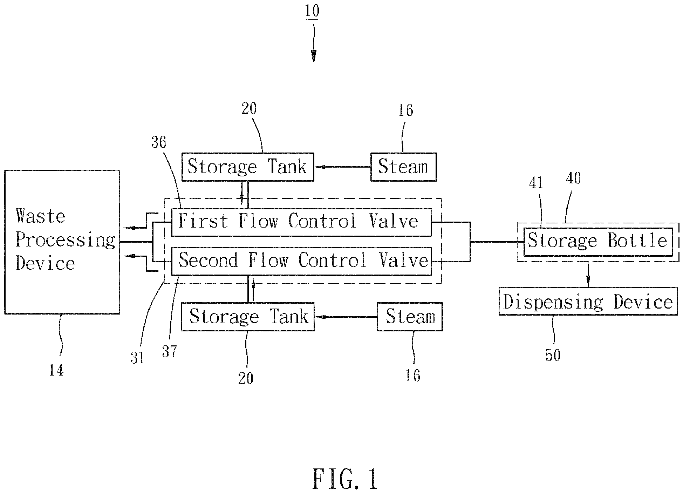

Referring to FIGS. 1, 4, and 5, an automatic dispensing apparatus 10 for dispensing a plant culture medium in a plurality of culture containers 12 in accordance with a preferred embodiment of the present invention is formed of two storage tanks 20, a control device 30, a filling device 40, and a dispensing device 50. The detailed descriptions and operations of these elements as well as their interrelations are recited in the respective paragraphs as follows.

As shown in FIG. 1-4, the control device 30 includes a switcher 31, a first feeding tube 32, a second feeding tube 33, a discharging tube 34, and a waste exhaust tube 35. The switcher 31 includes a first flow control valve 36 and a second flow control valve 37. Each of the first and second flow control valves 36 and 37 are arranged upside down and switchable between a first mode and a second mode. Each of the first and second feeding tubes 32 and 33 includes two ends, one of which is connected with one of the storage tanks 20 and the other is connected with one of the first and second flow control valves 36 and 37 of the switcher 31. The discharging tube 34 includes one end connected with the first and second flow control valves 36 and 37. The waste exhaust tube 35 includes two ends, one of which is connected with the first and second flow control valves 36 and 37 and the other is connected with a waste processing device 14. When the first flow control valve 36 is located at the first mode, the first feeding tube 32 communicates with the discharging tube 34 and does not communicate with the second feeding tube 33 and the waste exhaust tube 35. When the first flow control valve 36 is located at the second mode, the first feeding tube 32 communicates with the waste exhaust tube 35 and does not communicate with the second feeding tube 33 and the discharging tube 34. Similarly, when the second flow control valve 37 is located at the first mode, the second feeding tube 33 communicates the discharging tube 34 and does not communicate with the first feeding tube 32 and the waste exhaust tube 35. When the second flow control valve 37 is located at the second mode, the second feeding tube 33 communicates with the waste exhaust tube 35 and does not communicate with the first feeding tube 32 and the discharging tube 34.

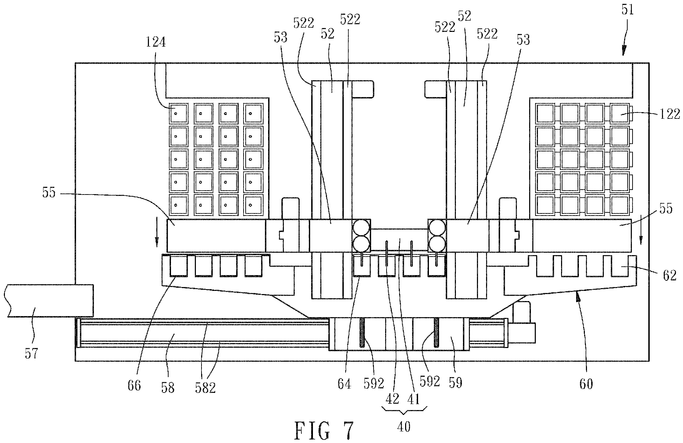

The filling device 40 includes a storage bottle 41 and a plurality of nozzles 42. As shown in FIGS. 1 and 7, the storage bottle 41 is connected with the discharging tube 34 for storing the plant culture medium outputted from the feeding tube 34. Each of the nozzles 42 includes one end connected with the storage bottle 41 for spraying the plant culture medium.

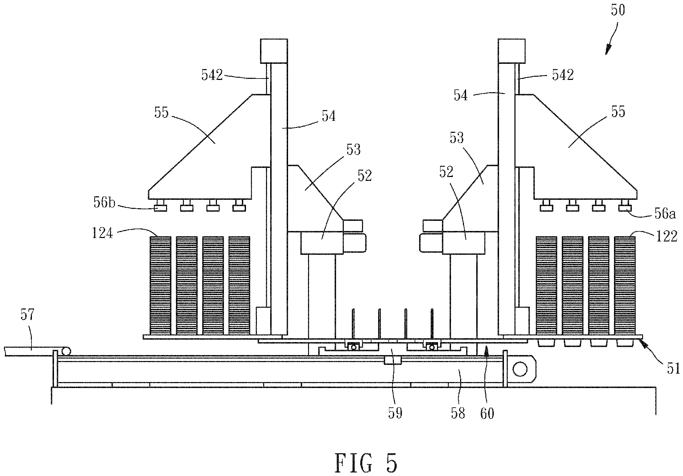

As shown in FIGS. 5 and 7, the dispensing device 50 is mounted adjacent to the filling device 40 and includes a container carrier 51, two X-axis platforms 52, two X-axis slide bases 53, two standing posts 54, two Z-axis slide bases 55, and two container catchers 56a and 56b. The two X-axis platforms 52 are apposed adjacent to the container carrier 51 and each include two X-axis slide rails 522 parallel to each other. Each of the X-axis slide bases 53 is mounted to the corresponsive X-axis slide rail 522 of the X-axis platform 52. Each of the standing posts 54 is mounted to the corresponsive X-axis slide rails 53 and includes two Z-axis slide rails 542. Each of the Z-axis slide bases 55 is mounted to the corresponsive Z-axis slide rails 542 and located above the container carrier 51. Each of container catchers 56a and 56b is mounted to a bottom side of the corresponsive slide base 55 for catching a chassis 122 and a top cover 124 of the corresponsive culture container 12.

Referring to FIGS. 5 and 7 again, the dispensing device 50 further includes an accommodating platform 57, a Y-axis platform 58, a Y-axis slide base 59, and a conveying platform 60. The Y-axis platform 58 is located between the container carrier 51 and the accommodating platform 57 and includes two Y-axis slide rails 582 parallel to each other. The Y-axis slide base 59 is mounted to the Y-axis slide rail 582 of the Y-axis platform 58 and includes two X-axis lead screws 592 parallel to each other. The conveying platform 60 is mounted to the X-axis lead screw 592 of the Y-axis slide base 59 for reciprocating movement among the filling device 40, the container carrier 51, and the accommodating platform 57 via the Y-axis slide base 59 and for forward and backward movement relative to the container carrier 51 axially along the X-axis lead screw 592 of the Y-axis slide base 59.

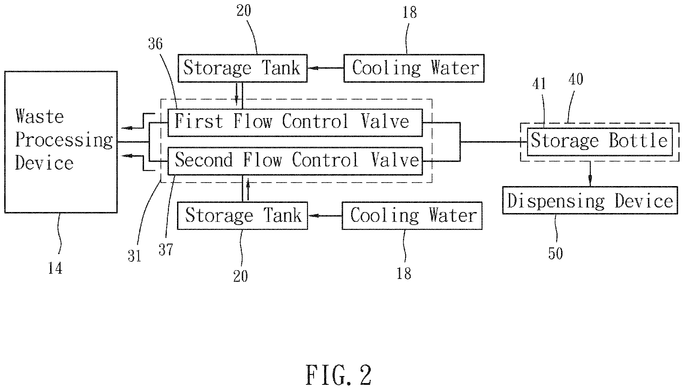

In actual operation, as shown in FIGS. 1, 2, and 4, the second flow control valve 37 of the switcher 31 is switched to the first mode, the first flow control valve 36 is switched to the second mode, and cleaning fluids, e.g. steam 16 and cooling water 18, are in order infused into the storage tank 20 connected with the first feeding tube 32 to pass through the first feeding tube 32 and exhaust through the waste exhaust tube 35 to reach the waste processing device 14 for sterilizing and cleaning the first feeding tube 32 and the storage tank 20 connected with the feeding tube 32. Referring to FIG. 2 again, the first flow control valve 36 is switched to the first mode, the second flow control valve 37 is switched to the second mode, and the stem 16 and the cooling water 18 are in order infused into the storage tank 20 connected with the second feeding tube 33 to pass through the second feeding tube 33 and exhaust through the waste exhaust tube 35 to reach the waste processing device 14 for sterilizing and cleaning the second feeding tube 33 and the storage tank 20 connected with the second feeding tube 33.

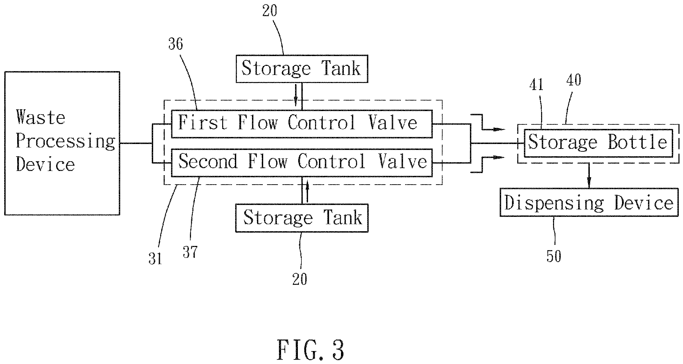

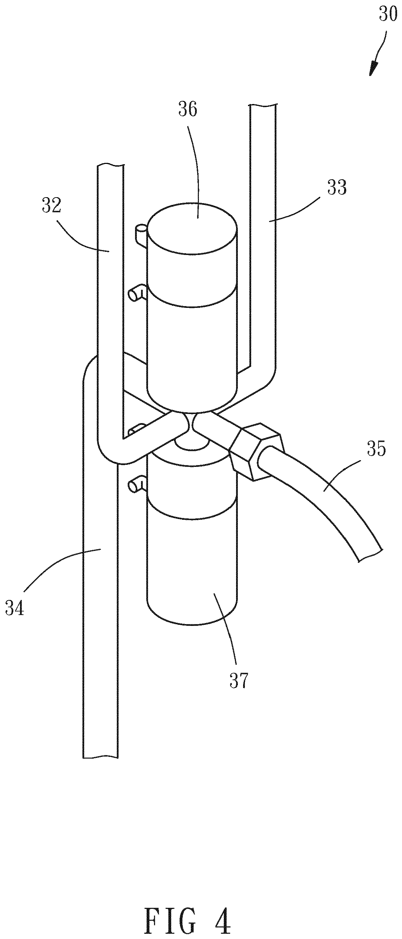

After the sterilization and the cleaning are done, as shown in FIGS. 3 and 4, the first flow control valve 36 remains at the first mode, the second flow control valve 37 remains at the second mode, and a first plant culture medium is infused into the first feeding tube 32 via the storage tank 20 connected with the first feeding tube 32 to enter the discharging tube 34 for filing the storage bottle 41 of the filling device 40 with the first plant culture medium. Next, the first flow control valve 36 is switched to the second mode, the second flow control valve 37 is switched to the first mode, and a second plant culture medium is infused into the second feeding tube 33 via the storage tank 20 connected with the second feeding tube 33 to enter the discharging tube 34 for filing the storage bottle 41 of the filling device 40 with the second plant culture medium.

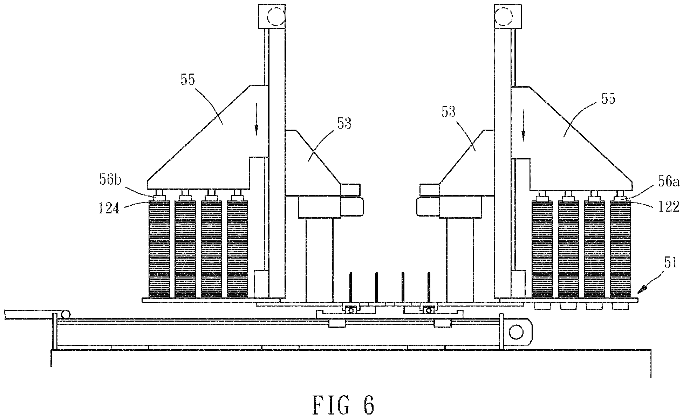

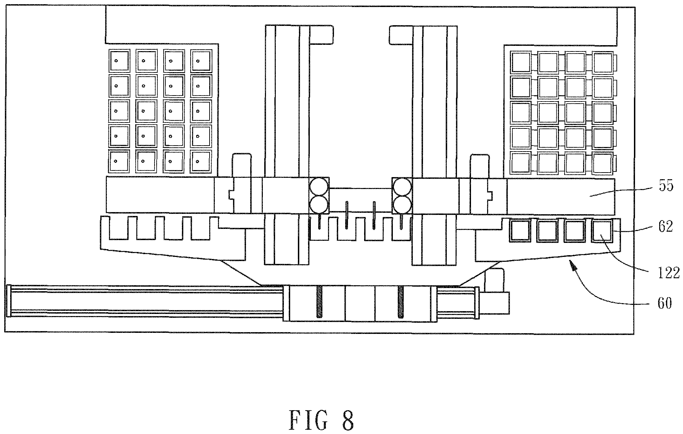

After the filing operations of the two plant culture media are completed, the dispensing device 50 continues to work. As shown in FIGS. 6 and 7, each of the container catchers 56a and 56b can catch the chassis 122 and the top cover 124 as the Z-axis slide base 55 is moved downward and then the X-axis slide base 53 makes the chassis 122 and the top cover 124 move forward to reach a position right over the conveying platform 60. Next, the container catcher 56a makes the chassis 122 engaged with a first engagement portion 62 as the X-axis slide base 55 is moved downward, as shown in FIG. 8. In the meantime, the conveying platform 60 can move the chassis 122 to a position in front of the filling device 40 via the Y-axis slide base 59 to make each of nozzles 42 spray adequate amount of the plant culture media onto the corresponsive chassis 122.

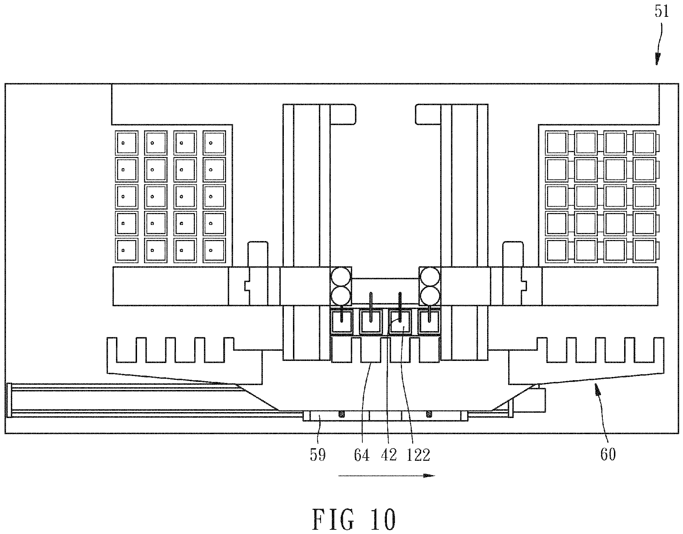

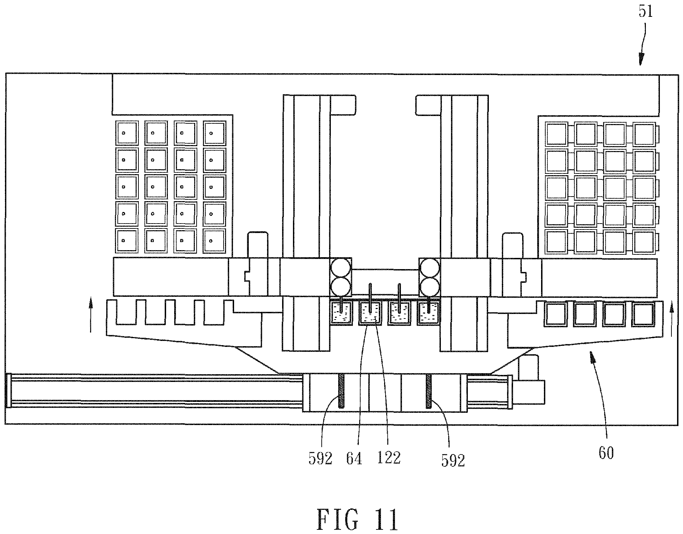

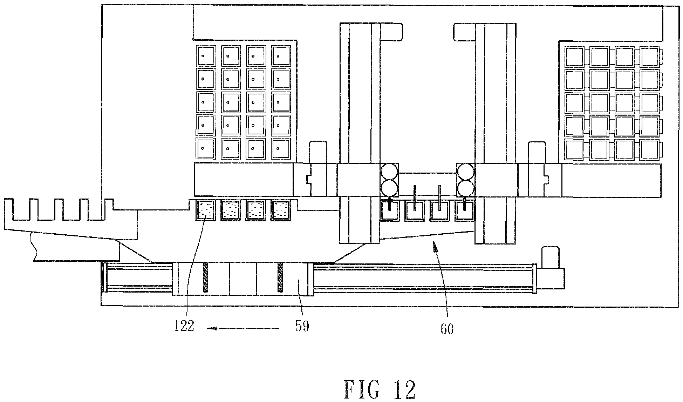

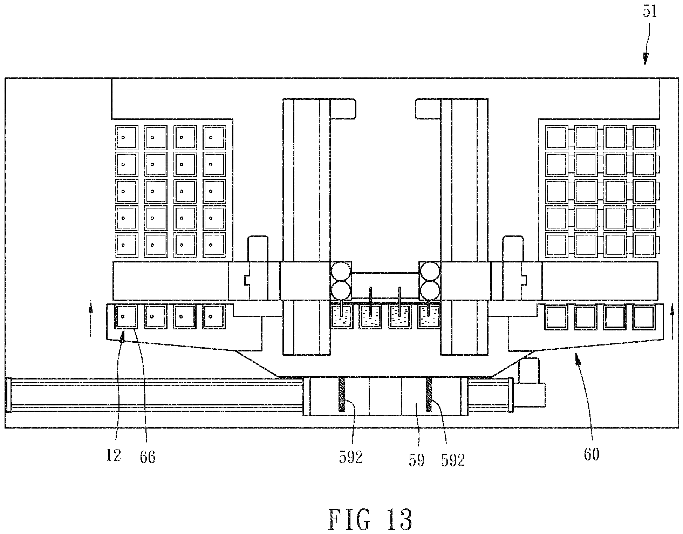

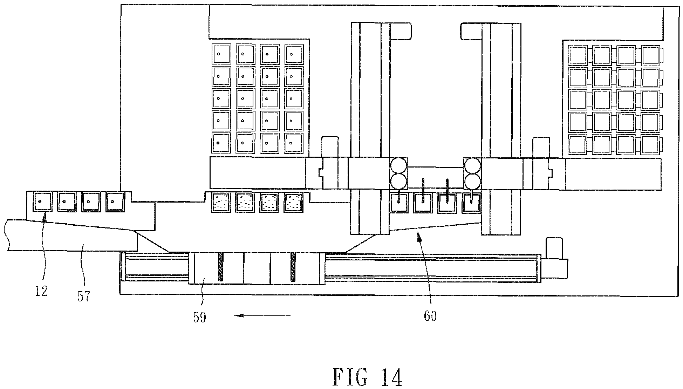

In the process of spraying the plant culture media, the conveying platform 60 can move away from the container carrier 51 via the X-axis lead screw 592, as shown in FIG. 10, to disengage the first engagement portion 62 from the corresponsive chassis 122. Once the conveying platform 60 is disengaged from the chassis 122, the conveying platform 60 returns to the initial position indicated in FIG. 8 via the Y-axis slide base 59 and then moves toward the container carrier 51 via the X-axis lead screw 592 to make the second engagement portion 64 engaged with the chassis 122, as shown in FIG. 11. Next, the conveying platform 60 conveys the chassis 122 to a position in front of the top cover 124 via the Y-axis slide base 59, as shown in FIG. 12, to make each of the chassis 122 correspond to one of the top covers 124 and meanwhile, the container catcher 56b makes the top cover 124 cap a top side of the corresponsive chassis 122 via the X-axis slide base 55 to complete the assembly of the container 12. In the meantime, the conveying platform 60 can move away from the container carrier 51 via the X-axis lead screw 592 to disengage the second engagement portion 64 from the chassis 122 and then return to the initial position indicated in FIG. 8 via the Y-axis slide base 59. Next, the conveying platform 60 moves toward the container carrier 51 via the X-axis lead screw 592 to make a third engagement portion 66 engaged with the chassis 122, as shown in FIG. 13. At last, the conveying platform 60 conveys the culture container 12 after the assembly to the accommodating platform 57 via the Y-axis slide base 59 to complete the conveying operation, as shown in FIG. 14.

In conclusion, the automatic conveying apparatus 10 of the present invention can apply the filling and conveying operations to a variety of plant culture media and proceed to sterilize and clean the storage tank and the associated filling tubes by means of the switcher 31 after all of the filling and dispensing operations are completed, thus reducing the labor cost, boosting the operational efficiency, and enhancing the filling stability.

## Claims

1. An automatic dispensing apparatus (10) for dispensing a plant culture medium in at least one culture container (12), being **characterized in that** the automatic dispensing apparatus (10) comprises:
two storage tanks (20) for storing the plant culture medium;
a control device (30) having a switcher (31), a first feeding tube (32), a second feeding tube (33), a discharging tube (34), and a waste exhaust tube (35), the first and second feeding tubes (32,33) connected between the switcher (31) and one of the storage tank (20), the discharging tube (34) having an end connected with the switcher (31), the waste exhaust tube (35) having an end connected with the switcher (31); when the switcher (31) is located at a first mode, the first feeding tube (32), the second feeding tube (33), and the waste exhaust tube (35) do not communicate with one another and one of the first and second feeding tubes (32,33) communicates with the discharging tube (34); when the switcher (31) is located at a second mode, the first feeding tube (32), the second feeding tube (33), and the discharging tube (34) do not communicate with one another and one of the first and second feeding tubes (32, 33) communicates with the waste exhaust tube (35);
a filling device (40) connected with the discharging tube (34) for filling the at least one culture container (12) with the plant culture medium; and
a dispensing device (50) mounted adjacent to the filling device (40) and having a container carrier (51), an accommodating platform (57), and a dispensing platform (60), the dispensing platform (60) being mounted in a way of reciprocating between the filling device (40), the container carrier (51), and the accommodating platform (57) for conveying the culture container (12) to the accommodating platform (57) from the filling device (40);

2. The automatic dispensing apparatus (10) as defined in claim 1, being **characterized in that** the switcher (31) comprises a first flow control valve (36) and a second flow control valve (37); when the first flow control valve (36) is located at the first mode, the first feeding tube (32) communicates with the discharging tube (34) and does not communicate with the second feeding tube (33) and the waste exhaust tube (35); when the first flow control valve (36) is located at the second mode, the first feeding tube (32) communicates with the waste exhaust tube (35) and does not communicate with the second feeding tube (33) and the discharging tube (34); when the second flow control valve (37) is located at the first mode, the second feeding tube (33) communicates with the discharging tube (34) and does not communicate with the first feeding tube (32) and the waste exhaust tube (35); when the second flow control valve (37) is located at the second mode, the second feeding tube (33) communicates with the waste exhaust tube (35) and does not communicate with the first feeding tube (32) and the discharging tube (34).

3. The automatic dispensing apparatus (10) as defined in claim 1, being **characterized in that** the filling device (40) comprises a storage bottle (20) and a nozzle (42), the storage bottle (20) being connected with the discharging tube (34), the nozzle (42) having an end connected with the storage bottle (20).

4. The automatic dispensing apparatus (10) as defined in claim 1, being **characterized in that** the dispensing device (50) further comprises an X-axis platform (52), an X-axis slide base (53), a standing post (54), a Z-axis slide base (55), and a container catcher (56a,56b), the X-axis platform (52) being mounted adjacent to the container carrier (51) and having two X-axis slide rails (5222), the X-axis slide base (53) being mounted to the X-axis slide rails (5222), the standing post (54) being mounted to the X-axis slide base (53) and having two Z-axis slide rails (542), the Z-axis slide base (55) being mounted to the Z-axis slide rails (542) and located above the container carrier (51), the container catcher (56a or 56b) being mounted to a bottom side of the Z-axis slide base (55).

5. The automatic dispensing apparatus (10) as defined in claim 1, being **characterized in that** the dispensing device (50) further comprises a Y-axis platform (58) and a Y-axis slide base (59), the Y-axis platform (58) being located between the container carrier (51) and the accommodating platform (57) and having a Y-axis slide rail (582), the Y-axis slide base (59) being mounted to the Y-axis slide rail (582) and having an X-axis lead screw (592), the conveying platform (60) being mounted to the X-axis lead screw (592).

## Patentansprüche

1. Automatische Ausgabevorrichtung (10) zum Ausgeben eines Pflanzenkulturmediums in wenigstens einen Kulturbehälter (12), **dadurch gekennzeichnet, dass** die automatische Ausgabevorrichtung (10) umfasst:
zwei Speichertanks (20) zum Speichern des Pflanzenkutturmediums;
eine Steuervorrichtung (30) mit einem Schalter (31), einer ersten Zuführröhre (32), einer zweiten Zuführröhre (33), einer Auslassröhre (34), sowie einer Abfallauslassröhre (35), die erste und zweite Zufuhrröhren (32,33) sind zwischen dem Schalter (31) und einem der Speichertanks (20) verbunden, ein Ende der Auslassröhre (34) ist mit dem Schalter (31) verbunden, ein Ende der Abfallauslassröhre (35) ist mit dem Schalter (31) verbunden; wenn sich der Schalter (31) in einem ersten Modus befindet, stehen die erste Zuführröhre (32), die zweite Zuführröhre (33) und die Abfallauslassröhre (35) miteinander nicht in Verbindung und eine der ersten und zweiten Zuführröhren (32,33) steht mit der Auslassröhre (34) in Verbindung; wenn sich der Schalter (31) in einem zweiten Modus befindet, stehen die erste Zuführröhre (32), die zweite Zuführröhre (33) und die Auslassröhre (34) miteinander nicht in Verbindung und eine der ersten und zweiten Zuführröhren (32, 33) steht mit der Abfallauslassröhre (35) in Verbindung;
eine Füllvorrichtung (40), die mit der Auslassröhre (34) verbunden ist, um den wenigstens einen Kulturbehälter (12) mit dem Pflanzenkulturmedium zu füllen; und
eine Ausgabevorrichtung (50), die angrenzend an die Füllvorrichtung (40) angeordnet ist und einen Behälterträger (51), eine Aufnahmeplattform (57), sowie eine Ausgabeplattform (60) aufweist, die Ausgabeplattform (60) ist derart angeordnet, dass sie sich zwischen der Füllvorrichtung (40), dem Behälterträger (51) und der Aufnahmeplattform (57) hin- und her bewegt werden kann, um den Kulturbehälter (12) zu der Aufnahmeplattform (57) von der Füllvorrichtung (40) zu transportieren.

2. Automatische Ausgabevorrichtung (10) nach Anspruch 1, **dadurch gekennzeichnet, dass** der Schalter (31) ein erstes Flusssteuerungsventil (36) und ein zweites Flusssteuerungsventil (37) umfasst; wenn sich das erste Flusssteuerungsventil (36) in dem ersten Modus befindet, steht die erste Zuführröhre (32) mit der Auslassröhre (34) in Verbindung und steht nicht mit der zweiten Zuführröhre (33) und der Abfallauslassröhre (35) in Verbindung; wenn sich das erste Flusssteuerungsventil (36) in dem zweiten Modus befindet, steht die erste Zuführröhre (32) mit der Abfallauslassröhre (35) in Verbindung und steht nicht mit der zweiten Zuführröhre (33) und der Auslassröhre (34) in Verbindung; wenn sich das zweite Flusssteuerungsventil (37) in dem ersten Modus befindet, steht die zweite Zuführröhre (33) mit der Auslassröhre (34) in Verbindung und steht nicht mit der ersten Zuführröhre (32) und der Abfallauslassröhre (35) in Verbindung; wenn sich das zweite Flusssteuerungsventil (37) in dem zweiten Modus befindet, steht die zweite Zuführröhre (33) mit der Abfallauslassröhre (35) in Verbindung und steht nicht mit der ersten Zuführröhre (32) und der Auslassröhre (34) in Verbindung.

3. Automatische Ausgabevorrichtung (10) nach Anspruch 1, **dadurch gekennzeichnet, dass** die Füllvorrichtung (40) eine Speicherflasche (20) und eine Düse (42) umfasst, die Speicherflasche (20) ist mit der Auslassröhre (34) verbunden und ein Ende der Düse (42) ist mit der Speicherflasche (20) verbunden.

4. Automatische Ausgabevorrichtung (10) nach Anspruch 1, **dadurch gekennzeichnet, dass** die Ausgabevorrichtung (50) ferner eine X-Achsenplattform (52), eine X-Achsen-Gleitbasis (53), eine Standsäule (54), eine Z-Achsen-Gleitbasis (55) und einen Behälterfänger (56a,56b) umfasst, die X-Achsenplattform (52) ist angrenzend an den Behälterträger (51) angeordnet und weist zwei X-Achsen-Gleitschienen (5222) auf, die X-Achsen-Gleitbasis (53) ist an den X-Achsen-Gleitschienen (5222) angeordnet, die Standsäule (54) ist an der X-Achsen-Gleitbasis (53) angeordnet und weist zwei Z-Achsen-Gleitschienen (542) auf, die Z-Achsen-Gleitbasis (55) ist an den Z-Achsen-Gleitschienen (542) angeordnet und befindet sich über dem Behälterträger (51), der Behälterfänger (56a oder 56b) ist an einer Unterseite der Z-Achsen-Gleitbasis (55) angeordnet.

5. Automatische Ausgabevorrichtung (10) nach Anspruch 1, **dadurch gekennzeichnet, dass** die Ausgabevorrichtung (50) ferner eine Y-Achsenplattform (58) und eine Y-Achsen-Gleitbasis (59) umfasst, die Y-Achsenplattform (58) befindet sich zwischen dem Behälterträger (51) und der Aufnahmeplattform (57) und weist eine a Y-Achsen-Gleitschiene (582) auf, die Y-Achsen-Gleitbasis (59) ist an der Y-Achsen-Gleitschiene (582) angeordnet und weist eine X-Achsen-Führungsschraube (592) auf, die Transportplattform (60) ist an der X-Achsen-Führungsschraube (592) angeordnet.

## Revendications

1. Distributeur automatique (10) pour la distribution d'un milieu de culture végétale dans au moins un récipient de culture (12), **caractérisé en ce que** le distributeur automatique (10) comprend :
deux réservoirs de stockage (20) destinés à stocker le milieu de culture végétale ;
un dispositif de commande (30) présentant un commutateur (31), un premier tube d'alimentation (32), un deuxième tube d'alimentation (33), un tube de décharge (34) et un tube d'évacuation de déchets (35), les premier et deuxième tubes d'alimentation (32, 33) étant reliés entre le commutateur (31) et l'un des réservoirs de stockage (20), le tube de décharge (34) présentant une extrémité reliée au commutateur (31), le tube d'évacuation de déchets (35) présentant une extrémité reliée au commutateur (31) ; dans lequel, lorsque le commutateur (31) se trouve dans un premier mode, le premier tube d'alimentation (32), le deuxième tube d'alimentation (33) et le tube d'évacuation de déchets (35) ne communiquent pas entre eux et l'un parmi les premier et deuxième tubes d'alimentation (32, 33) communique avec le tube de décharge (34) ; dans lequel, lorsque le commutateur (31) se trouve dans un deuxième mode, le premier tube d'alimentation (32), le deuxième tube d'alimentation (33) et le tube de décharge (34) ne communiquent pas entre eux et l'un parmi les premier et deuxième tubes d'alimentation (32, 33) communique avec le tube d'évacuation de déchets (35) ;
un dispositif de remplissage (40) relié au tube de décharge (34) pour remplir l'au moins un récipient de culture (12) avec le milieu de culture végétale ; et un dispositif de distribution (50) monté à côté du dispositif de remplissage (40) et présentant un support de récipient (51), une plateforme d'accueil (57) et une plateforme de distribution (60), la plateforme de distribution (60) étant montée de manière à effectuer un va-et-vient entre le dispositif de remplissage (40), le support de récipient (51) et la plateforme d'accueil (57) pour transporter le récipient de culture (12) vers la plateforme d'accueil (57) à partir du dispositif de remplissage (40).

2. Distributeur automatique (10) selon la revendication 1, **caractérisé en ce que** le commutateur (31) comprend une première soupape de commande d'écoulement (36) et une deuxième soupape de commande d'écoulement (37) ; dans lequel, lorsque la première soupape de commande d'écoulement (36) se trouve dans le premier mode, le premier tube d'alimentation (32) communique avec le tube de décharge (34) et ne communique pas avec le deuxième tube d'alimentation (33) et le tube d'évacuation de déchets (35) ; dans lequel, lorsque la première soupape de commande d'écoulement (36) se trouve dans le deuxième mode, le premier tube d'alimentation (32) communique avec le tube d'évacuation de déchets (35) et ne communique pas avec le deuxième tube d'alimentation (33) et le tube de décharge (34) ; dans lequel, lorsque la deuxième soupape de commande d'écoulement (37) se trouve dans le premier mode, le deuxième tube d'alimentation (33) communique avec le tube de décharge (34) et ne communique pas avec le premier tube d'alimentation (32) et le tube d'évacuation de déchets (35) ; dans lequel, lorsque la deuxième soupape de commande d'écoulement (37) se trouve dans le deuxième mode, le deuxième tube d'alimentation (33) communique avec le tube d'évacuation de déchets (35) et ne communique pas avec le premier tube d'alimentation (32) et le tube de décharge (34).

3. Distributeur automatique (10) selon la revendication 1, **caractérisé en ce que** le dispositif de remplissage (40) comprend une bouteille de stockage (20) et une buse (42), la bouteille de stockage (20) étant reliée au tube de décharge (34), la buse (42) présentant une extrémité reliée à la bouteille de stockage (20).

4. Distributeur automatique (10) selon la revendication 1, **caractérisé en ce que** le dispositif de distribution (50) comprend en outre une plateforme d'axe X (52), une base coulissante d'axe X (53), un montant fixe (54), une base coulissante d'axe Z (55), et un collecteur de récipient (56a, 56b), la plateforme d'axe X (52) étant montée à côté du support de récipient (51) et présentant deux rails coulissants d'axe X (5222), la base coulissante d'axe X (53) étant montée sur les rails coulissants d'axe X (5222), le montant fixe (54) étant monté sur la base coulissante d'axe X (53) et présentant deux rails coulissants d'axe Z (542), la base coulissante d'axe Z (55) étant montée sur les rails coulissants d'axe Z (542) et située au-dessus du support de récipient (51), le collecteur de récipient (56a ou 56b) étant monté sur un côté inférieur de la base coulissante d'axe Z (55).

5. Distributeur automatique (10) selon la revendication 1, **caractérisé en ce que** le dispositif de distribution (50) comprend en outre une plateforme d'axe Y (58) et une base coulissante d'axe Y (59), la plateforme d'axe Y (58) étant située entre le support de récipient (51) et la plateforme d'accueil (57) et présentant un rail coulissant d'axe Y (582), la base coulissante d'axe Y (59) étant montée sur le rail coulissant d'axe Y (582) et présentant une vis-mère d'axe X (592), la plateforme de transport (60) étant montée sur la vis-mère d'axe X (592).
